# EUROPEAN PATENT APPLICATION

(11) **EP 2 905 036 A1**
(43) Date of publication of application: **12.08.2015**
(21) Application number: 13843876.7
(22) Date of filing: 24.09.2013
(51) Int. Cl.: A61L 9/22, A61L 9/16, A61L 9/00

(54) **IONIZER**

(30) Priority: 02.10.2012 KR 20120109640
(71) Applicant: LG Electronics Inc., Seoul 150-721 (KR)
(72) Inventor: SUNG, Bongjo, Seoul 137-724 (KR); JANG, Jaesoo, Seoul 137-724 (KR); LEE, Junu, Seoul 137-724 (KR); SON, Ilna, Seoul 137-724 (KR); OH, Junggeun, Seoul 137-724 (KR)
(74) Representative: Vossius & Partner Patentanwälte Rechtsanwälte mbB
(86) International application number: PCT/KR2013/008526
(87) International publication number: WO 2014/054862

(57) **Abstract**

Provided is an ionizer. In an embodiment, concaves-convexes are formed on a surface of a substrate, one or more electrodes for generating a positive ion or a negative ion is formed, and the concaves-convexes are coated with photocatalyst. The electrode is formed on a surface opposite to the surface on which the concaves-convexes are formed, and two or more substrates are disposed in line at an interval while the electrode and the concave-convex face each other. The ionizer according to another embodiment includes a 3D substrate having a polygonal pillar shape in which polygonal planes are perforated; an electrode unit including one or more electrodes formed on at least one inner surface of pillar planes forming the polygonal pillar to generate a positive ion or a negative ion; and a coating part in which an inner surface facing the pillar plane with the electrode unit is coated with photocatalyst.

## Description

### BACKGROUND OF THE INVENTION

### Field of the invention

The present invention relates to an ionizer, and more particularly, to a structure of an ionizer for increasing an area of catalyst coating.

### Discussion of the Related Art

With the increase in the number of persons who have respiratory disease or show an allergic reaction due to environmental pollution which becomes severe, various products have been released, which are used to purify polluted air by generating positive ions or negative ions.

The negative ions mean a state in which molecules such as oxygen or nitrogen in the air have negative charges, since the negative ions are beneficial to the human body and further, effective even in removing dust and fragrance, a separate ionizer may be released, but an ion generating module may be added to an air conditioner or an air cleaner.

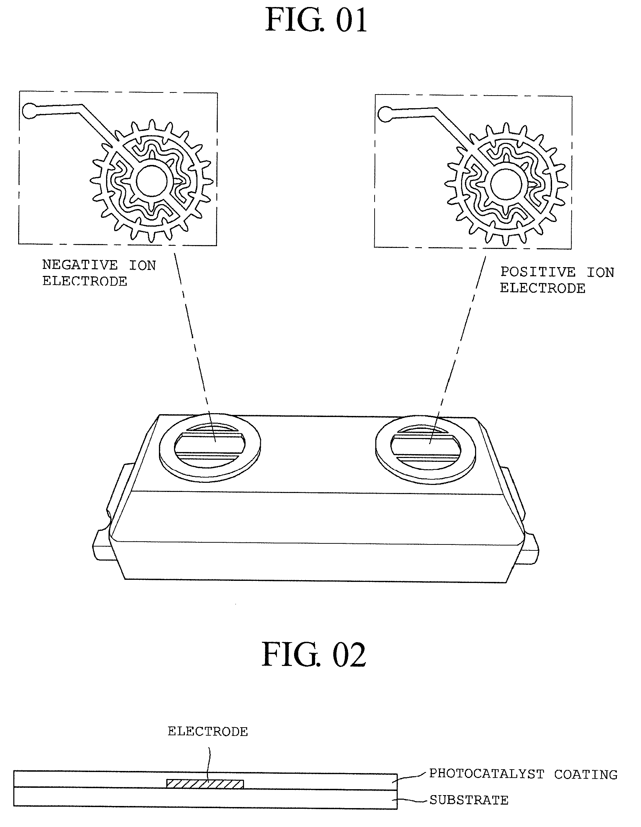

The ionizer has an outer shape illustrated in FIG. 1, an electrode unit constituted by a negative ion electrode for generating (-) ions and a positive ion electrode for generating (+) ions is formed on a rectangular substrate having a predetermined thickness, and when predetermined voltage is supplied to the electrode unit, the positive ions and the negative ions are together or selectively generated in the electrode unit.

In recent years, as illustrated in FIG. 2, the substrate is coated with photocatalyst capable of removing a harmful substance, for example, visible-ray catalyst or UV catalyst to improve performance such as removal of microorganism, deodorization, and the like of the ionizer and increase the amount of generated ions.

An area of the substrate needs to be increased to activate a catalytic reaction at the time of considering a principle in which the catalyst reacts, but the ion generator using the rectangular substrate, on which the electrode is just laid in the related art is limited in increasing the area of the substrate, and as a result, there is a limit in improving the microorganism or deodorization performance of the ion generator.

### Summary of the Invention

The present invention is contrived to solve the problem and an object of the present invention is to improve an air purification effect of an ionizer.

A detailed objet of the present invention is to provide a substrate structure that increases a reaction area of catalyst coating applied to an ionizer.

In accordance with an embodiment of the present invention, an ionizer includes: a substrate in which concaves-convexes are formed on at least one surface; and one or more electrodes for generating at least one of a positive ion and a negative ion, and the concaves-convexes are coated with photocatalyst.

The electrode may be formed on the concaves-convexes and the photocatalyst may be coated on the electrode may be coated with the photocatalyst.

The electrode may be formed on a surface opposite to the surface on which the concaves-convexes are formed, and two or more substrates may be disposed in line at an interval while the electrode and the concave-convex face each other.

In accordance with another embodiment of the present invention, an ionizer includes: a 3D substrate having a polygonal pillar shape in which polygonal planes facing each other are perforated; an electrode unit including one or more electrodes formed on at least one inner surface of pillar planes forming the polygonal pillar to generate at least one of a positive ion and a negative ion; and a coating part in which an inner surface facing the pillar plane with the electrode unit is coated with photocatalyst.

Multiple 3D substrates may be disposed to be connected.

The 3D substrate may be any one of a quadrangular pillar, a hexagonal pillar, and an octagonal pillar and in the 3D substrate having the hexagonal pillar shape, the electrode unit and the coating part may be alternately disposed.

A concave-convex may be formed on the inner surface of the pillar plane with the coating part.

The polygonal pillar may be disposed so that the perforated polygonal planes may be vertical to a direction in which an air current flows.

In accordance with yet another embodiment of the present invention, an ionizer includes: a case; an electrode unit including one or more electrode formed on one surface of the substrate to generate at least one of a positive ion and a negative ion; and a coating part in which inner surfaces of pillars for supporting the case or inner surfaces of grills for keeping an interval among the pillars are coated with photocatalyst.

The inner surfaces may be concave-convex and the convex-convex may be coated with the photocatalyst.

In accordance with still another embodiment of the present invention, an ionizer includes: a case; a rectangular substrate; an electrode unit including one or more electrode formed on one surface of the substrate to generate at least one of a positive ion and a negative ion; and a coating part coated with photocatalyst in a mesh form and located between the case and the substrate.

Accordingly, a coating area of catalyst increases, and as a result, a catalyst reaction is activated and polluted material dissolution and organic material dissolution performance is improved.

### Brief Description of the Drawings

FIG. 1 illustrates an outer shape and an electrode of an ionizer in the related art;
FIG. 2 illustrates an electrode substrate coated with catalyst;
FIG. 3 illustrates one surface of a substrate coated with catalyst, which is concave and convex according to an embodiment of the present invention;
FIG. 4 illustrates a polygonal pillar substrate according to another embodiment of the present invention;
FIG. 5 illustrates a lattice shaped substrate in which a plurality of quadrangular pillar substrate are connected according to another embodiment of the present invention;
FIG. 6 illustrates that two or more substrates, in which photocatalyst is coated on a surface opposite to a surface on which the electrode is formed, are disposed at a predetermined interval according to another embodiment of the present invention;
FIG. 7 illustrates a pillar or a grill coated with the photocatalyst according to another embodiment of the present invention; and
FIG. 8 illustrates that a coating part coated with photocatalyst in a mesh form is located in a space between an electrode substrate and a case according to yet another embodiment of the present invention.

### Detailed Description of the Embodiments

Hereinafter, an ionizer according to the present invention will be described in detail with reference to the accompanying drawings.

TiO₂ or photocatalyst similar thereto performs a photocatalyst reaction with visible rays or ultraviolet rays to generate OH or O₂, thereby removing a polluted material. A larger area is advantageously coated with the photocatalyst in order to increase the photocatalyst reaction area.

Accordingly, the present invention proposes a structure in which photocatalyst may be coated on a large area and an ionizer to which the structure is applied.

An ionizer according to an embodiment of the present invention may be configured to include a substrate, an electrode unit in which one or more electrodes for generating at least one of a positive ion and a negative ion are formed at a partial area of the substrate, a coating part coated with photocatalyst, a fan for forming an air current in order to discharge the ion generated from the electrode unit to the outside or introduce outdoor air, and a power supply unit for supplying operating power to the electrode unit and the fan.

When the ionizer is mounted on an air conditioner, an air cleaner, and the like in a module form, only the electrode unit, the coating part, and the substrate are manufactured in the module form and the fan and the power supply unit equipped in the air conditioner or the air embodiment may be used.

When high-voltage power is applied to the electrode of the electrode unit from the power supply unit, at least one of the negative ion and the positive ion may be generated and a predetermined quantity of ultraviolet rays may be additionally emitted. The photocatalyst of the coating part reacts to the ultraviolet rays emitted from the electrode unit to dissolve and remove polluted materials included in surrounding air.

In the case of the substrate of the ionizer in the related art, the electrode is formed on the substrate of which a rectangular surface is even and the top of the electrode is coated with the photocatalyst, and as a result, an area of the photocatalyst for dissolving the polluted materials is limited by the size of the rectangular substrate.

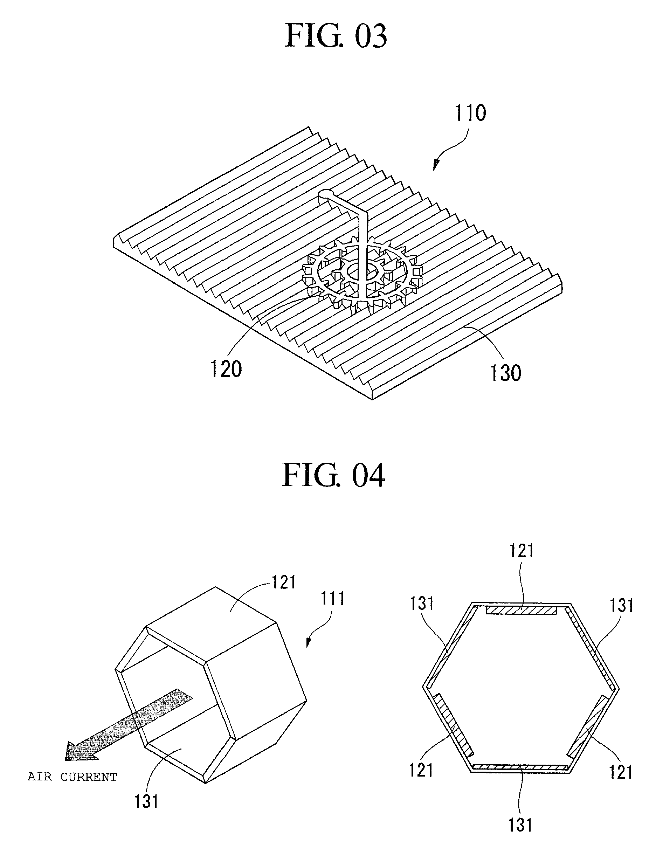

In the embodiment of the present invention, as illustrated in FIG. 3, a concave-convex is formed on at least one surface in the substrate 110, and the electrode unit 120 is thus disposed thereon and further, coated with the photocatalyst to form the coating part 130. Since a cross-sectional area of the surface is larger than that of the even substrate owing to the concave-convex shape, an area which is coated with the photocatalyst and reacts to the air may be increased.

A gap between the concaves-convexes or the width of a convex portion may be determined by considering the size of the electrode, a close attachment degree at which the electrode positioned on the substrate is fixed, and the like.

In another embodiment of the present invention, the substrate may be manufactured in not a plane shape but a 3D shape in which each of the electrode unit with the electrode and the coating part coated with the photocatalyst forms one surface and the respective surfaces face each other.

FIG. 4 illustrates a hexagonal pillar as one example of a 3D-shaped substrate. Electrode units 121 are formed three planes among six rectangular planes of a hexagonal pillar substrate 111, coating parts 131 are formed on three planes facing the electrode unit 121, and two hexagonal planes are perforated so as for the air to pass. The electrode units 121 and the coating parts 131 are preferably formed not outside but inside the pillar plane forming the pillar. Further, the electrode units 121 and the coating parts 131 may be not contiguous but alternately disposed on six rectangular pillar planes of the hexagonal pillar. The 3D-shaped substrate is not limited to the hexagonal pillar and may also adopt even polygonal pillars such as a quadrangular pillar, an octagonal pillar, and the like.

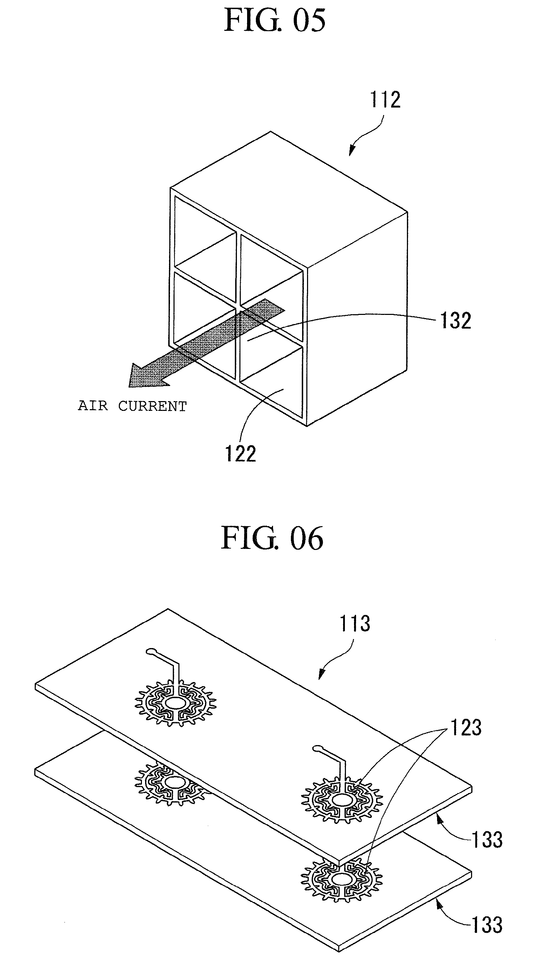

In another embodiment of the present invention, as illustrated in FIG. 5, a 3D substrate may be formed by connecting and attaching a plurality of polygonal pillars of FIG. 4. Only one plane of two pillar planes attached side by side is left, and as a result, neighboring polygonal pillars may share the corresponding pillar plane with each other. FIG. 5 illustrates a lattice-shaped substrate 112 formed by connecting four quadrangular pillars. An electrode unit 122 and a coating part 132 may be formed on an internal lattice plane through which an air current generated by a fan 40 will pass to face each other. A honeycombed substrate may be formed by connecting multiple hexagonal pillars and a substrate may be formed by alternately connecting the octagonal pillar and the quadrangular pillar.

Further, multiple polygonal pillars are connected symmetrically or asymmetrically to correspond to various space sizes.

In the embodiment of FIGS. 4 and 5, preferably, the pillar planes of the polygonal pillar are disposed in parallel to a direction in which the air current generated by the fan flows (alternatively, perforated polygonal planes are disposed vertical to the flow direction of the air current) to allow the air current to smoothly flow through the perforated polygonal planes.

In another embodiment of the present invention, the rectangular substrate in the related art is used as it is and as illustrated in FIG. 6, two or more substrates 113 are disposed in line at a predetermined interval and a rear surface of a plane on which the electrode is formed is coated with the photocatalyst to react to ultraviolet rays generated from an electrode on a plane opposite thereto.

The substrate having the electrode unit on one surface and the coating part on the opposite surface in the embodiment of Fig. 6 may be used on the pillar plane shared at the time of connecting multiple polygonal pillars as illustrated in FIG. 5.

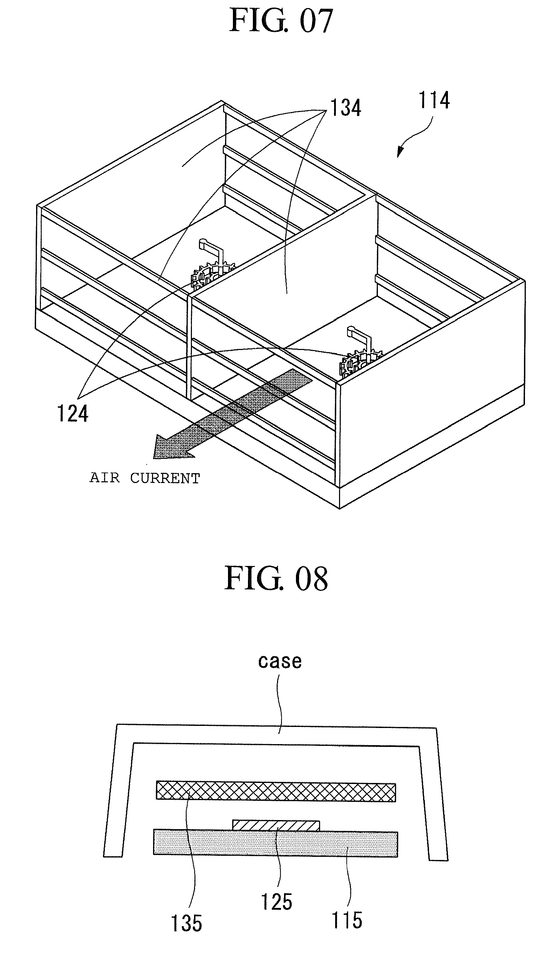

In another embodiment of the present invention, as illustrated in FIG. 7, a rectangular substrate 114 with an electrode unit 124 is used and a coating part 134 coated with the photocatalyst is formed on inner surfaces of pillars for supporting a case of an ionizer module or grills for keeping an interval among the pillars to increase a catalyst reaction area.

A concave-convex substrate on which the coating part coated with the photocatalyst is formed in the embodiment of Fig. 3 may be applied to the surface coated with the photocatalyst in the embodiments of FIGS. 4 to 7.

In yet another embodiment of the present invention, as illustrated in FIG. 8, a mesh coating part 135 coated with the photocatalyst is disposed at an upper side of a rectangular substrate 115 with an electrode unit 125 and between the electrode and the case of the ionizer module to increase the catalyst reaction area. That is, a space between the substrate 115 and the case is secured and the mesh-shaped coating part 135 is disposed so that the ions generated by the electrode unit 125 is easily mixed with the air current generated by the fan 40, and as a result, the ion reacts to the ultraviolet rays generated from the electrode unit 124 to easily dissolve the polluted materials included in the air current.

As described above, a catalyst reaction is activated by coating the large area with the photocatalyst to easily oxidize and dissolve the polluted materials in the air or dissolve more organic compounds.

A preferred embodiment of the present invention described above is disclosed for an exemplary purpose and modifications, changes, substitutions, or additions of various other embodiments can be hereinafter made by those skilled in the art within the technical spirit and the technical scope of the present invention disclosed in the appended claims.

## Claims

1. An ionizer comprising:
a substrate in which concaves-convexes are formed on at least one surface; and
one or more electrodes for generating at least one of a positive ion and a negative ion,
wherein the concaves-convexes are coated with photocatalyst.

2. The ionizer of claim 1, wherein the electrode is formed on the concaves-convexes and the photocatalyst is coated on the electrode.

3. The ionizer of claim 1, wherein:
the electrode is formed on a surface opposite to the surface on which the concaves-convexes are formed, and
two or more substrates are disposed in line at an interval while the electrode and the concave-convex face each other.

4. An ionizer comprising:
a 3D substrate having a polygonal pillar shape in which polygonal planes facing each other are perforated;
an electrode unit including one or more electrodes formed on at least one inner surface of pillar planes forming the polygonal pillar to generate at least one of a positive ion and a negative ion; and
a coating part in which an inner surface facing the pillar plane with the electrode unit is coated with photocatalyst.

5. The ionizer of claim 4, wherein multiple 3D substrates are disposed to be connected.

6. The ionizer of claim 4 or 5, wherein the 3D substrate is any one of a quadrangular pillar, a hexagonal pillar, and an octagonal pillar.

7. The ionizer of claim 6, wherein in the 3D substrate having the hexagonal pillar, the electrode unit and the coating part are alternately disposed.

8. The ionizer of claim 4 or 5, wherein a concave-convex is formed on the inner surface of the pillar plane with the coating part.

9. The ionizer of claim 4 or 5, wherein the polygonal pillar is disposed so that the perforated polygonal planes are vertical to a direction in which an air current flows.

10. An ionizer comprising:
a case;
a rectangular substrate;
an electrode unit including one or more electrode formed on one surface of the substrate to generate at least one of a positive ion and a negative ion; and
a coating part in which inner surfaces of pillars for supporting the case or inner surfaces of grills for keeping an interval among the pillars are coated with photocatalyst.

11. The ionizer of claim 10, wherein the inner surfaces are concave-convex and the convex-convex is coated with the photocatalyst.

12. The ionizer comprising:
a case;
a rectangular substrate;
an electrode unit including one or more electrode formed on one surface of the substrate to generate at least one of a positive ion and a negative ion; and
a coating part coated with photocatalyst in a mesh form and located between the case and the substrate.
